# EUROPEAN PATENT APPLICATION

(11) **EP 4 074 826 A1**
(43) Date of publication of application: **19.10.2022**
(21) Application number: 20899772.6
(22) Date of filing: 27.11.2020
(51) Int. Cl.: C12N 15/113, C12N 15/63, A61K 31/713, A61K 31/7105, A61P 25/14

(54) **SIRNA FOR INHIBITING HTT GENE EXPRESSION, AND PRECURSOR AND APPLICATION THEREOF**

(30) Priority: 13.12.2019 CN 201911283398
(71) Applicant: EXORNA BIOSCIENCE (NANJING) CO. LTD., Nanjing, Jiangsu 210031 (CN); EXORNA BIOSCIENCE (SHANGHAI) CO. LTD., Shanghai 201103 (CN)
(72) Inventor: CHEN, Xi, Nanjing, Jiangsu 210000 (CN); ZHANG, Li, Nanjing, Jiangsu 210000 (CN); PEI, Zhong, Nanjing, Jiangsu 210000 (CN); WU, Tengteng, Nanjing, Jiangsu 210000 (CN); LIU, Jin, Nanjing, Jiangsu 210000 (CN); YU, Mengchao, Nanjing, Jiangsu 210000 (CN); XIA, Yugui, Nanjing, Jiangsu 210000 (CN); TU, Sheng, Nanjing, Jiangsu 210000 (CN); LIN, Qiaofang, Nanjing, Jiangsu 210000 (CN)
(74) Representative: Laine IP Oy
(86) International application number: PCT/CN2020/132256
(87) International publication number: WO 2021/115141

(57) **Abstract**

The present invention provides a siRNA for inhibiting HTT gene expression and a precursor thereof, an expression vector comprising the siRNA or the precursor thereof, application thereof in preparation of a drug for treating Huntington's disease, and a pharmaceutical formulation and pharmaceutical composition containing the siRNA, the precursor or the expression vector. The siRNA is selected from the following: SEQ ID NO:1, SEQ ID NO:2, SEQ ID NO:3, SEQ ID NO:4, SEQ ID NO.:5, or a combination thereof.

Sequence Listing

<110>  NANJING YOUZHIYUAN PHARMACEUTICAL TECHNOLOGY GO., LTD.

<120>  SIRNA FOR INHIBITING HTT GENE EXPRESSION, AND PRECURSOR THEREOF AND
APPLICATION THEREOF

<130>  P2020-2291

<150>  CN201911283398.3
<151>  2019-12-13

<160>  10

<170>  PatentIn version 3.5

<210>  1
<211>  21
<212>  RNA
<213>  artificial sequence

<400>  1
ugacucugcg ucaucacugc a                                                21


<210>  2
<211>  21
<212>  RNA
<213>  artificial sequence

<400>  2
uaacuuuguu gaggcauucg u                                                21


<210>  3
<211>  21
<212>  RNA
<213>  artificial sequence

<400>  3
aaccuuggaa gauuagaauc c                                                21


<210>  4
<211>  20
<212>  RNA
<213>  artificial sequence

<400>  4
guagcugaaa guucuuucuu                                                  20


<210>  5
<211>  19
<212>  RNA
<213>  artificial sequence

<400>  5
uugguagcug aaaguucuu                                                   19


<210>  6
<211>  67

<212>  DNA
<213>  artificial sequence

<400>  6
aattcgtgac tctgcgtcat cactgcagtt ttggccactg actgactgca gtgaacgcag      60

agtcaca                                                                67


<210>  7
<211>  67
<212>  DNA
<213>  artificial sequence

<400>  7
aattcgtaac tttgttgagg cattcgtgtt ttggccactg actgacacga atgccaacaa      60

agttaca                                                                67


<210>  8
<211>  67
<212>  DNA
<213>  artificial sequence

<400>  8
aattcgaacc ttggaagatt agaatccgtt ttggccactg actgacggat tctacttcca      60

aggttca                                                                67


<210>  9
<211>  65
<212>  DNA
<213>  artificial sequence

<400>  9
aattcggtag ctgaaagttc tttcttgttt tggccactga ctgacaagaa agatttcagc      60

tacca                                                                  65


<210>  10
<211>  65
<212>  DNA
<213>  artificial sequence

<400>  10
aattcgttgg tagctgaaag ttcttgtttt ggccactgac tgacaagaac tttcagctac      60

caaca                                                                  65

## Description

### Technical Field

The present invention belongs to the field of biopharmaceuticals, and specifically relates to siRNAs that inhibit HTT gene expression and precursors thereof, and applications thereof.

### Background Technology

Huntington's disease (HD) is an autosomal dominant neurodegenerative disorder characterized by movement disorders. HD's main symptom is uncontrolled large limb movements, accompanied by cognitive impairment and psychiatric abnormalities. HD is a fatal neurodegenerative disorder that most often begins in adulthood and results in death within 15-20 years of onset. The field still lacks of effective treatments for HD. Therefore, it is important to find a safe and effective treatment.

Current therapeutic approaches using ASO to reduce the level of HTT mRNA and inhibit its translation have achieved preliminary clinical success. However, intrathecal injection of ASO is not conducive to its distribution in deep brain tissue, and intrathecal injection is an invasive procedure, which limits its clinical.

Therefore, there is an urgent need in the field to develop a siRNA capable of regulating HTT activity or expression.

### Summary of the Invention

The present invention provides a siRNA capable of regulating HTT activity or expression.

The first aspect of the present invention provides a precursor sequence having a structure from 5' to 3' as shown in formula (I): 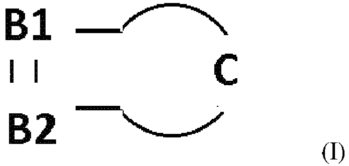
wherein B1 is a desired first ribonucleic acid sequence, wherein said first ribonucleic acid sequence comprises a HTT siRNA sense-strand sequence,
B2 is a sequence substantially or completely complementary to B1, and B2 is not complementary to C,
C is a stem-loop sequence;
wherein said HTT siRNA sense-strand sequence has a nucleotide sequence selected from the group consisting of: SEQ ID NO: 1, SEQ ID NO: 2, SEQ ID NO: 3, SEQ ID NO: 4, SEQ ID NO.: 5, or a combination thereof.

In another preferable embodiment, said precursor sequence has a sequence as shown in any of SEQ ID NO.:6-10.

The second aspect of the present invention provides a polynucleotide, wherein said polynucleotide is capable of being transcribed by a host to form the precursor sequence as described in the first aspect of the present invention.

The third aspect of the present invention provides an expression vector, wherein said expression vector contains the precursor sequence as described in the first aspect of the present invention or the polynucleotide as described in the second aspect of the present invention.

In another preferred embodiment, said expression vector includes a viral vector, a non-viral vector.

In another preferred embodiment, said expression vector is a plasmid.

In another preferred embodiment, said expression vector further contains a polynucleotide encoding a rabies virus surface glycoprotein short peptide (RVG peptide).

The fourth aspect of the present invention provides a pharmaceutical formulation, characterized in that said formulation comprises:
(a) an expression vector for expressing an siRNA that inhibits the expression of a HTT gene; and
(b) a pharmaceutically acceptable vector.

In another preferable embodiment, said expression vector comprises a polynucleotide expressing the precursor sequence as described in the first aspect of the present invention or a polynucleotide as described in the second aspect of the present invention, or expresses the precursor sequence as described in the first aspect of the present invention.

In another preferable embodiment, said expression vector expresses the precursor sequence as shown in formula I: 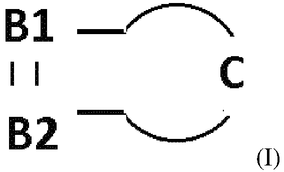
wherein B1 is a desired first ribonucleic acid sequence, wherein said first ribonucleic acid sequence comprises a HTT siRNA sense-strand sequence,
B2 is a sequence substantially or completely complementary to B1, and B2 is not complementary to C,
C is a stem-loop sequence;
wherein said HTT siRNA sense-strand sequence has a nucleotide sequence selected from the group consisting of: SEQ ID NO: 1, SEQ ID NO: 2, SEQ ID NO: 3, SEQ ID NO: 4, SEQ ID NO.: 5, or a combination thereof.

In another preferred embodiment, said expression vector further contains a polynucleotide encoding a rabies virus surface glycoprotein short peptide (RVG peptide).

In another preferred embodiment, said formulation is in liquid dosage form.

In another preferred embodiment, said formulation is in injection form.

In another preferred embodiment, said expression vector includes a plasmid.

In another preferred embodiment, said expression vector or plasmid comprises a promoter, a replication start point and a marker gene.

In another preferred embodiment, said expression vector contains an expression cassette expressing HTT siRNA.

In another preferred embodiment, said expression cassette (i.e. polynucleotide) is double-stranded and has the following structure:
promoter - attB1 - optional RVG - optional tag protein (e.g. Lamp2b) - 5' siRNA flanking region sequence - sequence shown in Formula I - 3' siRNA flanking region sequence - attB2.

In another preferred embodiment, said formulation is a liposomal or exosomal reagent.

The fifth aspect of the present invention provides a siRNA for inhibiting the expression of a HTT gene, characterized in that said siRNA has a sense strand nucleotide sequence selected from the group consisting of: SEQ ID NO: 1, 2, 3, 4, 5, or a combination thereof.

The sixth aspect of the present invention provides a pharmaceutical composition, characterized in that said pharmaceutical composition comprises the precursor sequence as described in the first aspect of the present invention, or an expression vector as described in the third aspect of the present invention, and a pharmaceutically acceptable vector.

In another preferred embodiment, said pharmaceutical composition comprises an HTT siRNA plasmid.

In another preferred embodiment, said pharmaceutical composition further comprises other drug for the treatment of Huntington's disease.

In another preferred embodiment, said other drug for the treatment of Huntington's disease is selected from the group consisting of: buprenorphine, ASO, or combinations thereof.

In another preferred embodiment, said pharmaceutical composition is an expression vector as described in the third aspect of the present invention, preferably, a plasmid containing the precursor sequence as described in the first aspect of the present invention.

In another preferred embodiment, said pharmaceutical composition is in the dosage form selecting from the group of: tablet, capsule, powder, pill, granule, syrup, solution, suspension, emulsion, suspension powder, injection, or powder injection.

In another preferred embodiment, said pharmaceutical composition is in the dosage form further selecting from the group of: a spray, aerosol, powdered aerosol, volatile liquid, topical solution, lotion, pour-on, application, baboo paste, cream, rubber paste, ointment, hard paste, paste, eye drop, nasal drop, ophthalmic ointment, gargle, sublingual tablet, or suppository.

In another preferred embodiment, said dosage form is an injection, preferably, an intravenous injection, an intraperitoneal injection.

The sixth aspect of the present invention provides a pharmaceutical composition, characterized in that said pharmaceutical composition comprises the precursor sequence as described in the first aspect of the present invention, or an expression vector as described in the third aspect of the present invention, and a pharmaceutically acceptable vector.

The seventh aspect of the present invention provides a use of the siRNA as described in the sixth aspect of the present invention, the precursor sequence as described in the first aspect of the present invention, or an expression vector as described in the third aspect of the present invention, characterized in that the use is for the preparation of a composition or formulation, said composition or formulation being used for (i) treating Huntington's disease.

In another preferred embodiment, said composition or formulation is further used for one or more purposes selected from the group consisting of:
(ii) inhibition of HTT expression in mammalian brain tissue; and/or
(iii) improving motor coordination in mammals.

In another preferred embodiment, said mammal includes a human or non-human mammal.

In another preferred embodiment, said non-human mammal includes a rodent (e.g., rat, rabbit), a primate (e.g., monkey).

In another preferred embodiment, said brain tissue includes the striatum, the cortex.

The eighth aspect of the present invention provides a method of treating Huntington's disease by administering a safe and effective dose of an expression vector as described in the third aspect of the present invention, a pharmaceutical formulation as described in the fourth aspect of the present invention, or a pharmaceutical composition as described in the sixth aspect of the present invention to a desired subject, thereby treating Huntington's disease.

In another preferred embodiment, the dose is 1-20 mg/kg, preferably, 5-10 mg/kg.

In another preferred embodiment, the frequency of administration is once every two days.

In another preferred embodiment, said administration includes oral, respiratory, injectable, transdermal, mucosal or luminal administration.

In another preferred embodiment, said administration comprises injecting a plasmid.

A ninth aspect of the present invention provides a method for (ii) inhibiting HTT expression in mammalian brain tissue; and/or (iii) improving motor coordination in a mammal, comprising:
administering a safe and effective dose of an expression vector as described in a third aspect of the present invention, a pharmaceutical formulation as described in a fourth aspect of the present invention, or a pharmaceutical composition as described in a sixth aspect of the present invention to a desired subject, thereby (ii) inhibiting the expression of HTT in mammalian brain tissue; and/or (iii) improving motor coordination in the mammal.

It should be understood that, within the scope of the present invention, each of the above technical features of the present invention and each of the technical features specifically described below (e.g., in embodiments) can be combined with each other so as to constitute new or preferred technical solutions.

### Brief Description of the Drawings

FIG. 1 is a diagram of the plasmid backbone of the present invention.
FIG. 2 is the cellular level of HTT siRNA expressed by the plasmid and inhibitory effect on HTT.
FIG. 3 is the disease treatment effect of HTT siRNA/RVG plasmid on transgenic mice BACHD.
Figure 3A shows the plasmid is administered by injection every two days and the mHTT protein expression level in the brain tissue of HD transgenic mice BACHD are measured for 3 months using western blot after seven injections. Figure 3B is the corresponding western blot quantification plot of Figure 3A, Figure 3C is the mRNA levels of mHTT detected in brain tissue using qpcr, and Figure 3D is the aggregation of mHTT in the striatum and cortex.
Figure 4 shows the disease treatment effect of HTT siRNA/RVG plasmids on transgenic mice N171-82Q.
Figure 4A shows the plasmid is administered by injection every two days and the HTT protein expression level in the brain tissue of HD transgenic mice N171-82Q are measured for 8 weeks using western blot after seven injections. Figure 4B is the corresponding western blot quantification plot of Figure 4A. Figure 4C shows the mHTT mRNA levels detected in brain tissue by using qpcr. Figure 4D is a graph of the effect of using RotaRod test to detect the balance coordination ability in mice.
Figure 5 shows the disease treatment effect of HTT siRNA/RVG plasmid on transgenic mice YAC HD.
Figure 5A shows the plasmid is administered by injection twice a week and the mHTT protein expression level in the brain tissue of HD transgenic mice YAC HD was detected using western blot after 8 weeks of injection. Figure 5B is the corresponding western quantification plot of Figure 5A. Figure 5C is the mHTT mRNA levels detected in brain tissues by using qpcr. Figure 5D is a graph of the effect of using RotaRod test to detect the balance coordination ability in mice. Figure 5E shows the aggregation of mHTT in striatum and cortex.

### Detailed Description of the Preferred Embodiments

After extensive and in-depth research, the inventors screened a large number of nucleic acid sequences and found for the first time that certain specific siRNAs have very high inhibitory ability for HTT.

Moreover, the present invention has provided for the first time a precursor siRNA that can express HTT siRNA efficiently. The precursor siRNA of the present invention can express siRNA efficiently after processed in the host cells, thus effectively avoiding the interference of the reverse complementary sequence of the target sequence on the function of the target sequence. It was demonstrated that the precursor siRNA of the present invention can effectively express HTT siRNA sequence in vivo and has more effective therapeutic effect on Huntington's disease. On this basis, the present inventors have completed the present invention.

### siRNAs and precursors thereof

As used herein, the term "siRNA" refers to a class of RNA molecules that are generated from progress of transcripts that can form siRNA precursors. Mature siRNAs typically have 18-26 nucleotides (nt) (more specifically about 19-22 nt), and may have other numbers of nucleotides. siRNAs are typically detectable by Northern blotting.

Human-derived siRNAs can be isolated from human cells. As used herein, "isolated" means that the substance is separated from its original environment (in the case of natural substances, the original environment is the natural environment). For example, polynucleotides and polypeptides in their natural state in living cells are not isolated and purified. The same polynucleotides or polypeptides are isolated and purified if they are separated from other substances present in their natural state.

It is noted that siRNA is generally produced by mimicking the miRNA generation mechanism, by which siRNA can be processed from precursor RNA (Pre-RNA). Said precursor RNA can be folded into a stable stem-loop (hairpin) structure, and said stem-loop structure is generally between 50-100 bp in length. Said precursor RNA may be folded into a stable stem-loop structure, with the stem-loop structure containing two sequences on either side of the stem that are substantially complementary. Said precursor RNA may be natural or synthetic.

In the present invention, said precursor siRNA is a synthetic precursor siRNA, and said precursor siRNA has the structure shown in formula I: 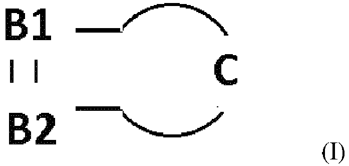
as a representative example, B1 is a HTT siRNA sense-strand sequence,
B2 is a sequence complementary (including substantially or completely) to B1,
C is a stem-loop sequence,
wherein the precursor siRNA as shown can be processed in the host to form HTT siRNA.

In the present invention, the precursor miRNAs forming HTT siRNAs can be sheared to generate siRNAs that regulate HTT genes, i.e., HTT siRNAs (e.g., SEQ ID NO.: 1, 2, 3, 4, 5):
SEQ ID NO 1: UGACUCUGCGUCAUCACUGCA
SEQ ID NO 2: UAACUUUGUUGAGGCAUUCGU
SEQ ID NO 3: AACCUUGGAAGAUUAGAAUCC
SEQ ID NO 4: GUAGCUGAAAGUUCUUUCUU
SEQ ID NO 5: UUGGUAGCUGAAAGUUCUUU.

The precursor RNA may be sheared to generate siRNA, and said siRNA may be substantially complementary to at least a portion of the sequence of the mRNA encoding the gene. In Formula I, B2 and B1 are substantially complementary. As used herein, "substantially complementary" means that the sequences of nucleotides are sufficiently complementary to interact in a predictable manner, such as forming a secondary structure (e.g., a stem-loop structure). Typically, two "substantially complementary" nucleotide sequences are at least 70% complementary to each other; preferably, at least 80% of the nucleotides are complementary; more preferably, at least 90% of the nucleotides are complementary; further preferably, at least 95% of the nucleotides are complementary; e.g., 98%, 99% or 100%. Generally, two sufficiently complementary molecules may have up to 40 mismatched nucleotides between them; preferably, have up to 30 mismatched nucleotides; more preferably, have up to 20 mismatched nucleotides; further preferably, have up to 10 mismatched nucleotides, such as having 1, 2, 3, 4, 5, 8, 11 mismatched nucleotides.

In a preferred embodiment of the present invention, the precursor of the present invention is as shown in any of SEQ ID NO.:6-10 below:

HTT-Si409sense5'-AATTCGTGACTCTGCGTCATCACTGCAGTTTTGGCCACTG
ACTGACTGCAGTGAACGCAGAGTCACA-3' (SEQ ID NO.:6)

HTT-Si449sense5'AATTCGTAACTTTGTTGAGGCATTCGTGTTTTGGCCACTG
ACTGACACGAATGCCAACAAAGTTACA-3' (SEQ ID NO.:7)

HTT-Si483sense5'-AATTCGAACCTTGGAAGATTAGAATCCGTTTTGGCCACT
GACTGACGGATTCTACTTCCAAGGTTCA-3' (SEQ ID NO.:8)

HTT-Si271sense5'-AATTCGGTAGCTGAAAGTTCTTTCTTGTTTTGGCCACTGA
CTGACAAGAAAGATTTCAGCTACCA-3' (SEQ ID NO.:9)

HTTSi-275-sense5'AATTCGTTGGTAGCTGAAAGTTCTTGTTTTGGCCACTGAC
TGACAAGAACTTTCAGCTACCAACA-3') (SEQ ID NO.:10).

As used in the present application, a "stem-loop" structure, also known as a "hairpin" structure, refers to a nucleotide molecule that can form a secondary structure comprising a double-stranded region (stem). The double-stranded region is formed by the two regions of the nucleotide molecule (located on the same molecule), on either side of the double-stranded portion. It also includes at least one "loop" structure comprising non-complementary nucleotide molecules, i.e., a single-stranded region. The double-stranded portion of the nucleotide may remain double-stranded even if the two regions of the nucleotide molecule are not fully complementary. For example, insertions, deletions, substitutions, etc. can result in non-complementarity of a small region, or result in the formation of a stem-loop structure or other form of secondary structure by the small region itself, however, the two regions can still be substantially complementary and interact in a predictable manner to form a double-stranded region of the stem-loop structure. Stem-loop structures are well known to those skilled in the art, and typically after obtaining a nucleic acid having a nucleotide sequence with a primary structure, those skilled in the art can determine whether the nucleic acid is capable of forming a stem-loop structure.

In the present invention, the "stem-loop structure" may be present at one end of the precursor siRNA of formula I, e.g., C forms a fixed terminal stem-loop structure due to the complementation of B1 and B2. The "stem-loop structure" may also exist inside the siRNA, for example, because B1 and B2 are not fully complementary to each other, resulting in an internal loop formed of bases of B1 or B2 that are not complementarily bound.

The siRNAs described in the present invention are: microRNAs of the family of siRNAs that inhibit HTT, said microRNAs of the family of siRNAs that inhibit HTT include: siRNAs that inhibit HTT or modified derivatives of siRNAs that inhibit HTT.

In a preferred embodiment of the present invention, the nucleotide sequence of the siRNA that inhibits HTT is shown in SEQ ID NO.:1-5 (corresponding to siRNA-1, siRNA-2, siRNA-3, siRNA-4, siRNA-5, respectively, in embodiments). Preferred sequences are SEQ ID NO.:2 and 5, and more preferably SEQ ID NO.:5.

The present invention also includes siRNA variants and derivatives. In addition, siRNA derivatives in the broad sense may also include siRNA variants. A person of ordinary skill in the art can modify the siRNA using general methods. Modifications includes (but not limited to): methylation modification, hydrocarbonyl modification, glycosylation modification (e.g., 2-methoxy-glycosyl modification, hydrocarbon-glycosyl modification, glycocyclic modification, etc.), nucleotide modification, peptide modification, lipid modification, halogen modification, nucleic acid modification (e.g. "TT" modification), etc.

### Polynucleotide constructs

According to the siRNA sequences provided by the present invention, polynucleotide constructs can be designed that can be processed to generate siRNAs that can affect the expression of the corresponding mRNA after being introduced, i.e. said polynucleotide constructs are capable of upregulating the amount of the corresponding siRNA in vivo. Thus, the present invention provides an isolated polynucleotide (construct), said polynucleotide (construct) can be transcribed by a human cell to be a precursor RNA, said precursor RNA can be sheared by human cells and expressed to be said siRNA.

As a preferred embodiment of the present invention, said polynucleotide constructs contain one or more structural units as shown in formula II:

Seq_{forward} - X - Seqᵣₑᵥₑᵣₛₑ (II)

In Formula II, the Seq_{forward} is a nucleotide sequence that can be expressed in a cell to be said siRNA for suppression of HTT, and Seqᵣₑᵥₑᵣₛₑ is a nucleotide sequence substantially complementary to Seq_{forward}; or, Seqᵣₑᵥₑᵣₛₑ is a nucleotide sequence that can be expressed in a cell as said siRNA, and Seq_{forward} is a nucleotide sequence substantially complementary to Seqᵣₑᵥₑᵣₛₑ; X is a spacer sequence disposed between Seq_{forward} and Seqᵣₑᵥₑᵣₛₑ, and said spacer sequence is not complementary to Seq_{forward} and Seqᵣₑᵥₑᵣₛₑ;
wherein each structural unit may express the same or different siRNAs.

The structure as shown in formula II, upon transfer into the cell, forms the secondary structure as shown in formula III: 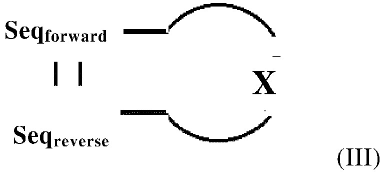 in Formula III, Seq_{forward}, Seqᵣₑᵥₑᵣₛₑ and X are defined as above.

∥ denotes the base complementary pairing relationship formed between Seq_{forward} and Seqᵣₑᵥₑᵣₛₑ.

Typically, the polynucleotide constructs described as above are included in an expression vector. Thus, the invention further provides a vector which contains said siRNA, or said polynucleotide construct. Said expression vector typically also contains a promoter, a replication start point and/or a marker gene, etc. Methods known to those skilled in the art can be used to construct the expression vectors required for the present invention. These methods include in vitro recombinant DNA techniques, DNA synthesis techniques, in vivo recombinant techniques, etc. Said expression vectors preferably comprise one or more selective marker genes to provide phenotypic traits such as caramycin, gentamicin, thaumatin, ampicillin resistance in the host cells selected for transformation.

### Pharmaceutical compositions and methods of administration

As used herein, the term "effective amount" or "effective dose" refers to an amount that is functional or active in humans and/or animals and is acceptable to humans and/or animals.

As used herein, the term "pharmaceutically acceptable" means a substance that is suitable for use in humans and/or mammals without undue adverse side effects (e.g., toxicity, irritation, and metabolic reactions), i.e., a substance with a reasonable benefit/risk ratio. The term "pharmaceutically acceptable vehicle" refers to a vehicle for the administration of a therapeutic agent, including various excipients and diluents.

The pharmaceutical compositions of the present invention contain a safe and effective amount of the active ingredient of the invention and a pharmaceutically acceptable carrier. Such carriers include (but are not limited to): saline, buffer, glucose, water, glycerol, ethanol, and combinations thereof. Usually the pharmaceutical formulation should match the route of administration, and the dosage forms of the pharmaceutical compositions of the present invention include injections, oral formulations (tablets, capsules, oral liquids), transdermal agents, and extended release agents. They are prepared, for example, with saline or aqueous solutions containing glucose and other excipients by conventional methods. Said pharmaceutical compositions are preferably manufactured under aseptic conditions.

The effective amount of the active ingredient described in the present invention may vary depending on the route of administration and the severity of the disease to be treated, etc. The selection of a preferred effective amount may be determined by one of ordinary skill in the art (e.g., by clinical trials) based on various factors. Said factors include, but are not limited to: the pharmacokinetic parameters of said active ingredient such as bioavailability, metabolism, half-life, etc.; the severity of the disease to be treated in the patient, the patient's body weight, the patient's immune status, the route of administration, etc. Generally, satisfactory results are obtained when the active ingredient of the present invention is given at a dose of about 0.00001mg-50mg/kg animal body weight per day (preferably 0.0001mg-10mg/kg animal body weight). For example, according to the exigencies of the therapeutic condition, several separate doses may be given daily, or the dose may be reduced proportionally.

The pharmaceutically acceptable carriers described in the present invention include (but are not limited to): water, saline, liposomes, lipids, micro particles, micro vesicles, exosomes, shedding vesicles, nanocapsules/nanoparticles), β-cyclodextriniclusion compound proteins, protein-antibody adducts, peptides, fibrils, nanogels, or combinations thereof. The choice of carrier should be matched to the route of administration, which are well known to those of ordinary skill in the art.

In the present invention, said expression vector may be administered directly to the subject, or said expression vector may be prepared in a pharmaceutical combination with a pharmaceutically acceptable carrier for administration. Said administration includes intravenous injection.

### Method of treatment

The present invention also provides a method of treating Huntington's disease, i.e., treating Huntington's disease by administering a safe and effective amount of an expression vector or pharmaceutical composition of the present invention to a desired subject.

The main advantages of the present invention include.
(a) The present invention is the first to develop siRNA sequences specifically designed for HTT, which can effectively inhibit HTT expression in brain tissue, and improve motor coordination, thus effectively treat Huntington's disease.
(b) The precursor siRNA of the present invention can effectively avoid overexpressing the reverse complementary sequence while overexpressing the target sequence, thus effectively avoiding the interference effect of the reverse complementary sequence of the target sequence on the function of the target sequence.
(c) By developing and designing a specific protein tag (RVG-Lamp2b), the siRNA expressed downstream of the tag can cross the blood-brain barrier smoothly and efficiently reach the brain tissue and exert its function.
(d) The drug of the present invention is administered by intravenous injection, which is less invasive and safer than intrathecal or intracranial injection.

The present invention is further described below with specific embodiments. It should be understood that these embodiments are intended to illustrate the invention only and are not intended to limit the scope of the invention. Experimental methods for which specific conditions are not indicated in the following embodiments generally follow conventional conditions, such as those described in Sambrook et al, Molecular Cloning: Laboratory Manual (New York: Cold Spring Harbor Laboratory Press, 1989), or as recommended by the manufacturer. Percentages and rations are by weight unless otherwise stated. Ingredients or instruments used in the embodiments of the invention are commercially available unless otherwise specified.

### General Methods

### I. Genotype identification

### 1. Main reagents and instruments

Reagents: Mouse genome extraction kit (Beyotime Biotechnology, China), Q5 high-fidelity DNA polymerase kit (NEB, USA), 100bp DNA ladder (NEB, USA), 5x DNA loading buffer (NEB, USA)

Instruments: water bath (Labnet, USA), PCR instrument (BIOMETRA, Germany), centrifuge (Eppendorf, Germany), electrophoresis instrument (BIO-RAD, USA), Nano Drop UV spectrophotometer (Thermo, USA), UV transmittance instrument (SANYO, Japan)

### 2, Main solution preparation

1X TAE electrophoresis buffer: 0.5M EDTA (pH8.0) 2ML, Tris base 4.84g, glacial acetic acid 1.142mL, deionized water filled up to 1L.

### 3, Genotype identification primer sequences and amplification procedure

BACHD mouse genotype identification primer sequences and amplification procedure:
Upstream primer: AGG TCG GTG CAG AGG CTC CTC
Downstream primer: CCG CTC AGG TTC TGC TTT TA
Step 1: 98°C 5min; Step 2: 98°C 10s; Step 3 65°C 20s; Step 4 72°C 20s; Step 5: Go to step 2 29 Cycles; Step 6: 72°C 10s; Step7 4°C Maintain
N171-82Q mouse genotype identification primers sequence and amplification procedure:
   Upstream primer: GTG GAT ACC CCC TCC CCC AGC CTA GAC C
   Downstream primer: GAA CTT TCA GCT ACC AAG AAA GAC CGT GT
   Step 1: 98°C 5min; Step 2: 98°C 20s; Step 3: 65°C 15s; Step 4: 68°C 10s; (repeat step 2 to step 4, annealing temperature decreased by 0.5 °C each cycle); Step 5: 98°C 15s; Step 6: 60°C 15s; Step 7: 72°C 10s; Step 8: Go to step 5 28 cycles; Step 9: 72°C 2min; Step 10: 4°C maintain
   YAC128 mouse genotype identification primer sequences and amplification procedure:
      Upstream primer: GGC TGA GGA AGC TGA GGA G
      Downstream primer: CCG CTC AGG TTC TGC TTT TA
      Step 1: 98°C 5min; Step 2: 98°C 20s; Step 3: 65°C 15s; Step 4: 68°C 10s; (repeat step 2 to step 4, annealing temperature decreased by 1.5°C each cycle); Step 5: 98°C 15s; Step 6: 60°C 15s; Step 7: 72°C 10s; Step 8: Go to step 5 28 cycles; Step 9: 72°C 2min; Step 10: 4°C maintain

### 4, Experimental methods and steps

### 4.1 Extraction of mouse genomic DNA

(1) After the transgenic mice are beyond lactation (3 weeks old), cut and obtain about 2-3mm of the tail of the mice, add 180µL of tissue lysate A and 20µL of proteinase K, incubate at 56°C for overnight until the tail of the mice is gelatinous.
(2) Add 200µL of Tissue Lysate B and mix thoroughly. Transfer to the separation column.
(3) Centrifuge at 12000 rpm for 1 min at room temperature and discard the waste solution.
(4) Add 500µL of Washing Solution A, centrifuge at 12000rpm at room temperature for 1min, and discard the waste solution.
(5) Add 500µL of Washing Solution B, centrifuge at 12000rpm for 1min at room temperature, and discard the waste solution.
(6) Transfer the column to a new 1.5mL EP tube, add 50µL of eluent and incubate for 2min at room temperature.
(7) Centrifuge at 12000 rpm for 1 min at room temperature, and the centrifuged liquid contains mouse genomic DNA.
(8) Quantify the concentration of mouse genomic DNA by Nano Drop UV spectrophotometer.

### 4.2 Mouse genotype identification

(1) Configure the PCR reaction system according to the following formula 5xQ5 reaction buffer 10ul 10mMdNTP mix lul 10µMPrimer Up 2.5u 10µMPrimer Down 2.5ul Template DNA 100ng Q5 DNA polymerase 0.5ul Deionized water filled up to 50ul
(2) Set up PCR reaction steps according to 3 conditions
(3) Preparation of agarose electrophoresis gel: This experiment uses a gel with a concentration of 1%, dissolve 0.5g of agar in 50 mL of 1×TAE solution to prepare the gel solution, and heat it in a microwave oven for 2min to dissolve it completely. Add 5 µL of Gel Red solution, mix well and pour into the gel-making tank while it is still hot, and insert the comb.
(4) After the gel cools and solidifies, pull out the comb and put the gel into the electrophoresis tank, with the end of the sample addition hole facing the cathode, and pour 1×TAE into the electrophoresis tank until it is not covered by the prepared agar gel.
(5) Add 10µL of 5XDNA Loading Buffer to the PCR product, blow and mix well, then add the sample to the spiked wells, and take another spiked well to add 100bp DNA Marker.
(6) Start electrophoresis at a constant pressure of 120V for 30 min.
(7) Remove the gel and place it on a UV transilluminator for imaging.

### II. Behavioral Testing.

All behavioral testing were performed from 9:00-11:00am. Mice were placed in the behavioral testing room in advance and acclimated for 1 hour before the start of the experiment. A light source of 60w white bulb was placed 2.4m above each behavioral device to be tested. After each test, the corresponding behavioral equipment was sprayed with 75% ethanol and wiped clean.

### 1. Fatigue RotaRod Test

In order to test the motor coordination of mice, we chose the Rotarod test. The procedure was as follows: the RotaRod Test was performed before the intervention, 2 weeks or 4 and 8 weeks after the treatment. The first day of the test was a training module in which the mice were allowed to run on a rotarod at a constant speed of 4 rpm for 5 min. The test was repeated 3 times at 30 min intervals to allow the mice to adapt to running on the rotarod. This is followed by a measurement module for 3 days. The rotarod will be accelerated uniformly from 4 rpm to 40 rpm within 3 min and the time that the mice can maintain an upright position on the rotarod will be recorded. The test will be repeated 3 times a day at 30min intervals.

### 2. Open field test

In order to measure the autonomic activity of mice, we chose the open field test. The apparatus for the open-field test was a closed box of 25x25x38 cm with a camera mounted on the top lid of the box. At the observation time point, mice were placed in the box, the lid was closed, and the video recording was started. The distance travelled by the mice in the box within 5 min was recorded.

### 3. Balance beam test

In order to measure the balance and limb coordination ability of the mice, we selected the balance beam test. 80 cm long, 12 mm wide, square cross-sectional wooden stick was used for this experiment. The bar was placed horizontally 50 cm above the table, with one end suspended and the mouse cage at one end. For the first 3 days of the experiment, the mice were placed on the suspended end of the stick with their heads facing the cage. The mice were urged to return to the cage by pushing their bodies, and this was repeated 3 times a day at intervals of >10 minutes. The mice were able to return to the cage on their own after 3 days of training without external stimulation. On the test day, each mouse was repeated 4 times with each interval greater than 10 minutes, and the average number of times the limb slipped off the wooden stick was recorded.

### III. Biochemical assay

### 1. Mice sampling

(1) Mice were anesthetized with sodium pentobarbital overdose and fixed properly, and the thoracic cavity was exposed with surgical instruments.
(2) Insert the perfusion needle into the left ventricle of the mouse, followed by cutting open the right atrium with ophthalmic scissors.
(3) Instill 100 mL of 0.1 M PBS (pre-chilled at 4°C).
(4) After completion of perfusion, sever the head and quickly remove the brain tissue.
(5) Surgically separate both sides of the brain tissue from the cortex and striatum on ice.

### 2. Protein quantification by Western Blot of mouse brain tissue

### 2.1 Main reagents and instruments

Reagents: RIPA Protein Extraction Solution (Thermo, USA), Phostop Phosphatase Inhibitor (Roche, Switzerland), PMSF (Invitrogen, USA), BCA Protein Quantification Kit (Thermo, USA), 4X SDS Loading Buffer (Dr.D., China), Pre-stained Protein Marker (Thermo, USA), SDS-MSF, and SDS-MSF. (Thermo, USA), SDS-PAGE kit (Biyuntian, China), PVDF membrane (Millipore, USA), mouse anti-HTT antibody (Millipore, USA), HRP-labeled sheep anti-mouse antibody (CST, USA), chemiluminescent solution (Millipore, USA)

Instruments: electrophoresis instrument (Bio-Rad, USA), chemiluminescence imager (GE, USA), horizontal rotary shaker (Labnet, USA), cryogenic centrifuge (Eppendorf, Germany), ultrasonic lysis instrument (SANYO, Japan), tissue grinder (Qiagen, Germany), enzyme marker (Thermo, USA)

### 2.2 Main solution preparation

(1) 10 × electrophoresis solution: distilled water volume to 1000 mL. glycine 144 g Tris base 30 g SDS 10 g
(2) 10 × transfer solution: distilled water volume to 1000 mL. glycine 144 g Tris base 30 g
(3) 1 × membrane transfer solution: distilled water to 1000 mL, ready to use, pre-chilled at 4°C. 10 × membrane transfer solution 100 mL methanol 200 mL
(4) 10 × TBS: distilled water volume to 1000 mL, adjust pH value to 7.6. Tris base 24.2g Nacl 80g
(5) 1 × TBST: distilled water volume to 1 L. 10 × TBS 100 mL Tween-20 1 mL
(6) 5% closed BSA: divided into 10mL tubes, stored at -20°C. 1 × TBST 100 mL BSA 5 g
(7) Secondary antibody diluent: 1 × TBST 100 mL BSA 1g
(8) 6% isolation gel: 5.3ml of ultrapure water, 30% Acr/Bic 2ml, 1.5 mol/L Tris-HCl (pH8.8) 2.5ml, 10% SDS 0.1ml, 10% AP 0.1ml, TEMED 0.008ml
(9) 5% gel concentrate: 2.85 ml of ultrapure water, 0.85 ml of 30% Acr/Bic, 1.25 ml of 0.5 mol/L Tris-HCl (pH 6.8), 0.05 ml of 10% SDS, 0.025 ml of 10% AP, 0.005 ml of TEMED

### 2.3 Extraction of brain tissue proteins from mice

(1) After perfusion, remove the brain tissue quickly, place it on ice, separate the cortex and striatum with microtweezers, put them in 1.5mL EP tubes, and snap-freeze them in liquid nitrogen.
(2) Add Phostop phosphatase inhibitor to the RIPA tissue lysate in a 100:1 ratio to the protease inhibitor PMSF according to the reagent instructions.
(3) Weigh the brain tissue to be extracted and add to the tissue lysate at a ratio of 100 mg/100 µL.
(4) Lysis by ultrasonic lysis in an ice bath for 5 s. Repeat 3-5 times until there is no visible tissue mass in the lysate.
(5) Let stand on ice for 30 min, centrifuge at 14000 rpm for 15 min at 4°C, and transfer the supernatant to a new EP tube for measurement.
(6) Determine the concentration of the extracted protein samples by BCA method, level the sample protein concentration to 5 µ g/ µ L, and dispense into 20 µ L/tube and store at -80°C.

### 2.4 SDS-PAGE protein gel electrophoresis (Western blot)

(1) Wash the glass plate and dry it in the oven at 37°C. The bottom edge of the glass plate was aligned and clamped in the glue dispensing device, and then the glass plate was stuck on the rack so that the bottom edge was pressed tightly in the sealing rubber strip to prevent glue leakage.
(2) Configure 6% separating glue, blow and mix it well, then slowly add the separator glue between the two glass plates along the side of the glass plate, then add isopropyl alcohol to press the glue, shake it 2-3 times left and right and leave it for 30min at room temperature, and wait for the separator glue to solidify.
(3) After the gel is solidified, pour off the isopropyl alcohol and absorb the excess liquid with filter paper, prepare 5% gel concentrate, blow and mix it, then slowly add it between the two glass plates along the side of the glass plate, then insert the pre-washed and dried comb between the two glass plates quickly, pay attention to avoid air bubbles, and leave it for 30 min at room temperature.
(4) Remove the sample to be tested from the -80°C refrigerator, thaw in an ice bath, add 4X SDS Loading Buffer at a ratio of 3:1, mix by blowing, boil for 10 min at 100°C, and then leave on ice for use.
(5) Clamp the glass plate in the electrophoresis clamp, pull out the comb and pour in 1× electrophoresis solution.
(6) Put on 8 µL (30 µg) of the protein sample to be detected and 8 µL of 170 kDa or 350 kDa protein marker.
(7) 80 V electrophoresis until the loading buffer of bromfen blue is run through the concentrated gel and compressed into a line, then constant pressure 120 V electrophoresis until the target protein is well separated.
(8) Transfer membrane: Cut 7cm × 6cm PVDF membrane, soak it thoroughly in methanol for 20s, then equilibrate it in 1X transfer solution for 5 min. Separate the glass plate, discard the concentrated gel, and place the separated gel on the lined filter paper. In a 1X transfer solution water bath, place the clear side of the clamp, sponge, two layers of filter paper, PVDF membrane, separator gel, two layers of filter paper, sponge, and the black side of the clamp in the order from positive to negative to create a transfer sandwich structure.
(9) Then put the clips into the transfer tank with the black side of the clips facing the black side of the tank and the transparent side of the clips facing the red side of the tank, add 1× transfer solution, turn wet on ice, and 300mA constant current for 180 min.
(10) Remove the PVDF membrane, rinse with 1X TBST for 1 min, and close the membrane with 5% BSA in a shaker at room temperature for 1h.
(11) After closure, cut out the target protein and the internal reference protein from the PVDF membrane according to the position of the protein marker bands and place them in the antibody incubation box, add the primary antibody diluted with 5% BSA and incubate overnight at 4°C in a shaker.
(12) After overnight incubation, the membranes were rinsed in 1X TBST for 3 times at room temperature for 5 min each time.
(13) Then add the secondary antibody diluted with 1% BSA and incubate for 1h at room temperature on a shaker.
(14) Rinse 3 times in 1X TBST with shaking at room temperature for 5 min each time.
(15) Configure the chemiluminescent solution according to the instructions.
(16) Soak the PVDF film in the chemiluminescent solution, remove and place it on the sample tray of the chemiluminescence instrument, blot the excess solution with filter paper and perform chemiluminescent imaging.

### Example 1: Construction of HTT siRNA/RVG plasmid and interference efficiency assay

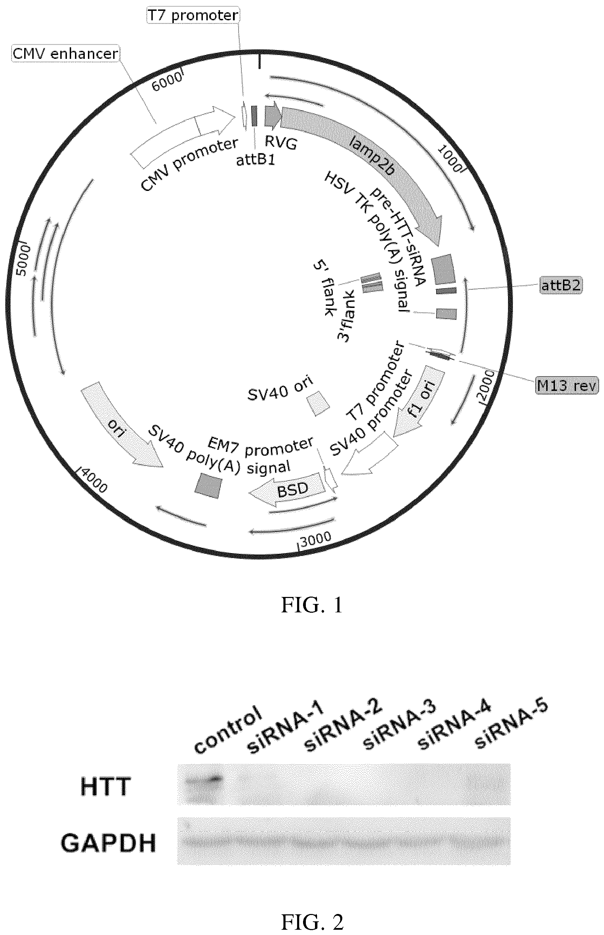

The control plasmid was treated using restriction endonuclease, and after recovering the linear vector, the combined RVG-HTT siRNA fragment was ligated to the vector using T4 ligase; the resulting ligated product was subjected to transformation experiments and coated on a resistance plate; the next day, a single clone was picked and sequenced to determine the correctness of the plasmid sequence. According to this method, four combinatorial fragments containing different combinatorial fragments targeting HTT were ligated into the vector to form plasmid molecules (plasmid structures are shown in Figure 1), named HTT si-1, HTT si-2, HTT si-3, HTT si-4, and HTT si-5, respectively.

The five plasmid molecules were transfected with human-derived neural cell line SHSY5Y at 60-70% confluence, and the expression levels of HTT in the cells were detected using western assay after 30 hours. The results showed that all five HTT si interfered with HTT protein with relatively high efficiency (as shown in Figure 2).

### Example 2: Inhibitory effect of plasmid-expressed siRNA on HTT in vivo

Taking HTT si-5 as an example, HTT si-5 was used for subsequent experiments, and the control plasmid and HTT siRNA/RVG plasmid were injected into the tail vein of 3-month-old HD transgenic mice BACHD at a dose of 5 mg/kg once every two days for a total of seven injections, and the mice were executed 24 hours after the last injection, and the brain tissues of the mice were taken for western blot experiments.

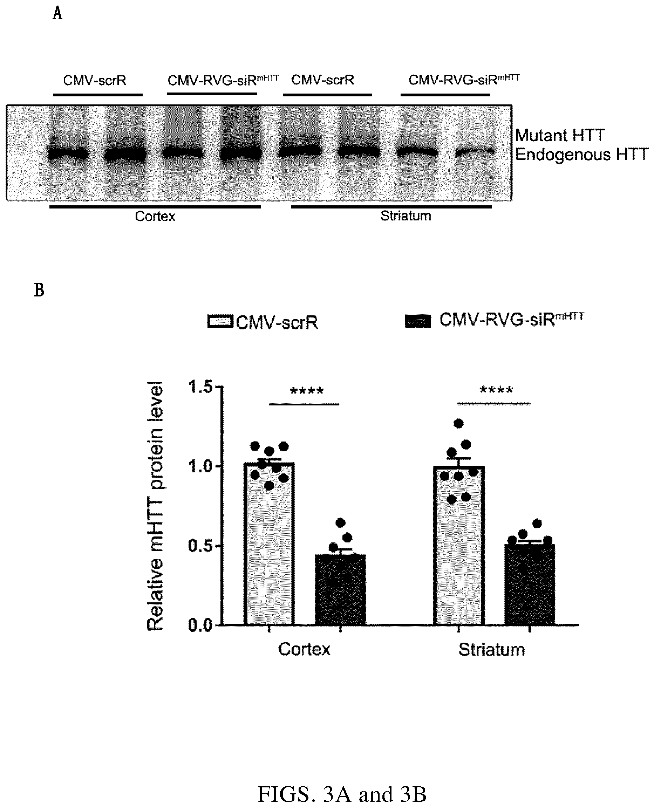

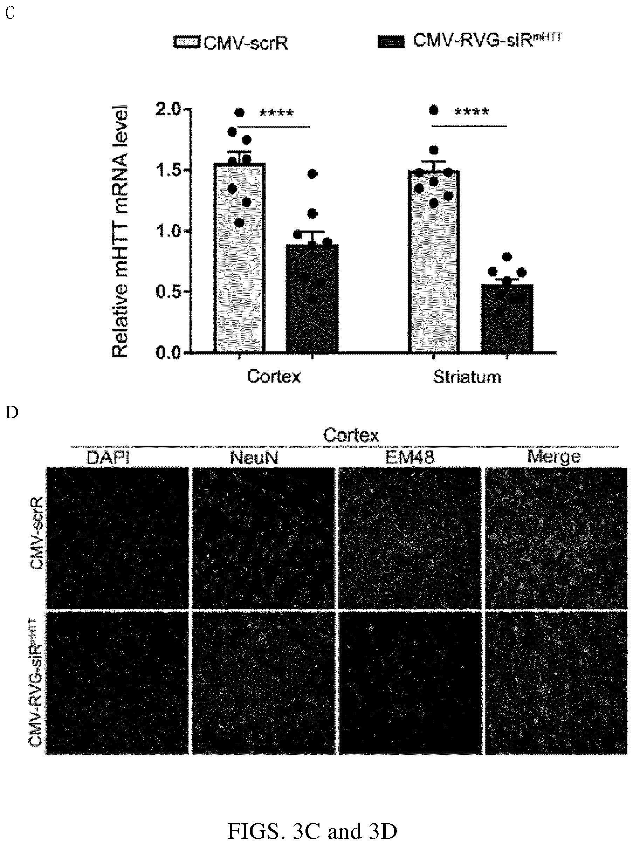

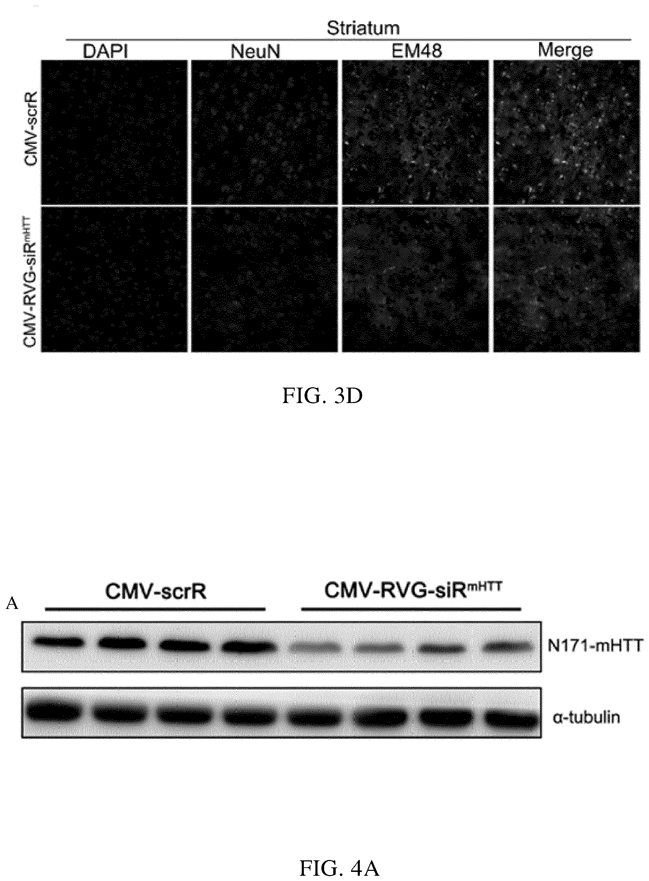

The results showed that HTT si-5 plasmid molecule could effectively reduce the protein and mRNA expression levels of mHTT in brain tissues in vivo (as shown in Figure 3, A-C). Reduction of mHTT mutant protein aggregation in the cortex and striatum (shown in Figure 3D, EM48 is the antibody that specifically binds to the aggregated mHTT).

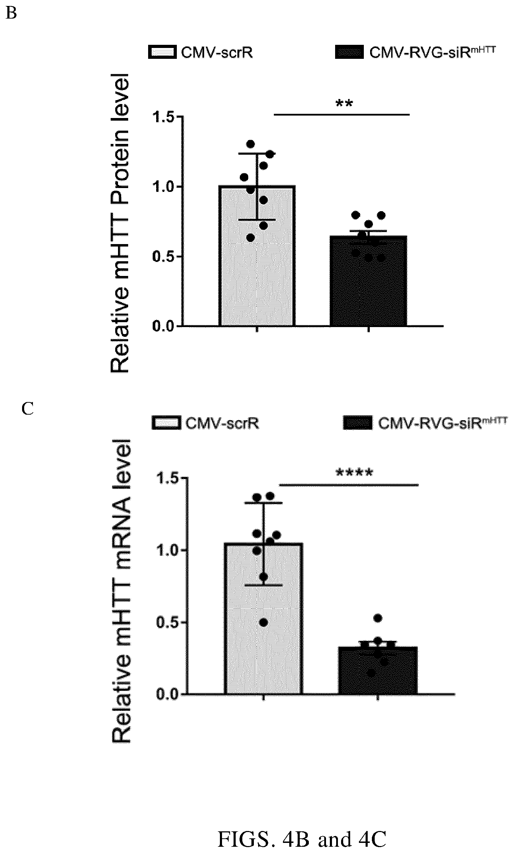

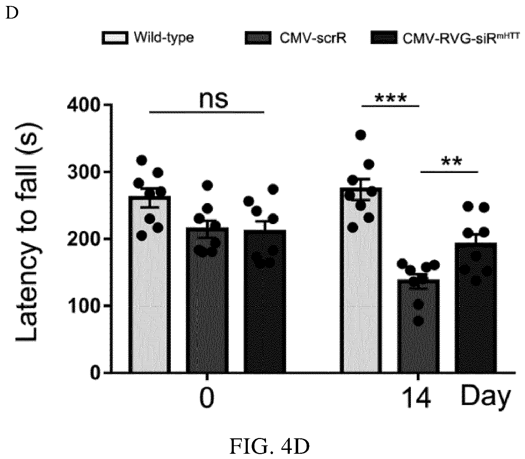

The 8-week HD transgenic mice N171-82Q were randomly divided into control and treatment groups, and the control plasmid and HTT si-5 plasmid were injected into the tail vein of N171-82Q mice at a dose of 5 mg/kg once every two days for a total of seven injections, and the mice were executed 24 hours after the last injection, and the brain tissues were taken for western blot experiments. The results showed that HTT si-5 plasmid molecule could effectively reduce the protein and mRNA expression levels of mHTT in brain tissues in vivo (as shown in Figure 4, A-C).

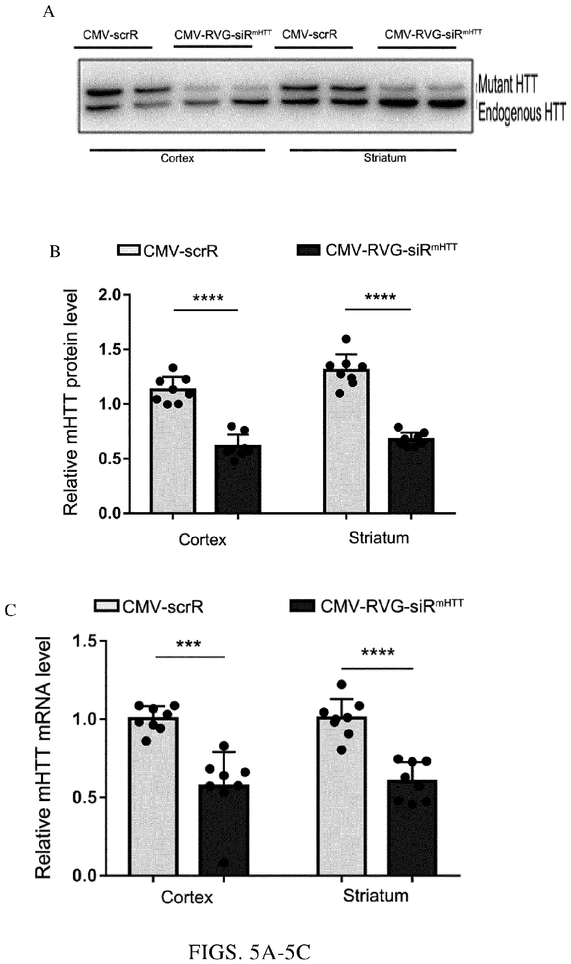

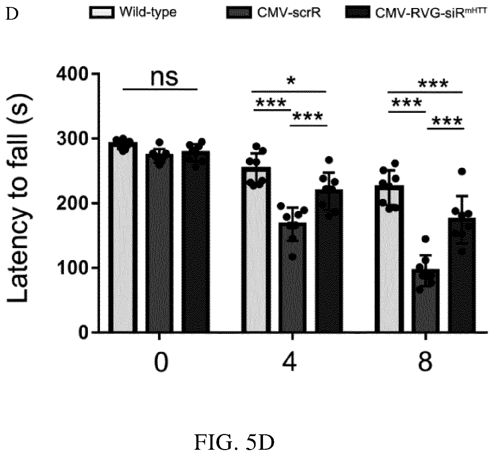

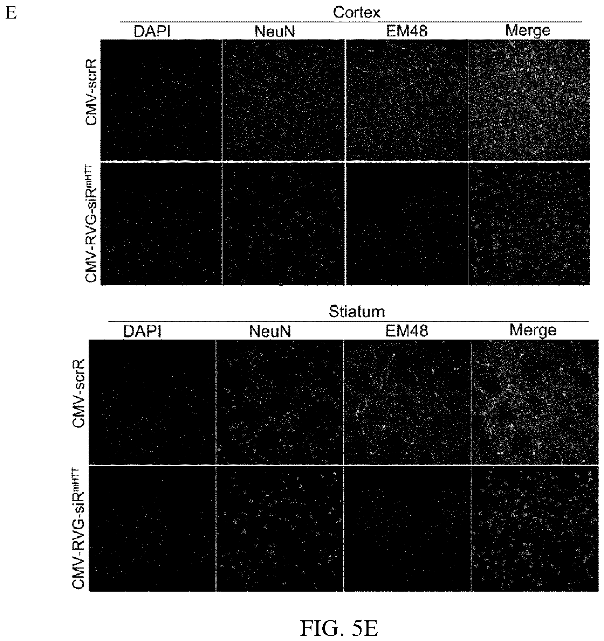

The 6-week-old HD transgenic mice YAC128 were randomly divided into control and treatment groups, and the control plasmid and HTT si-5 plasmid were injected into the tail vein of YAC128 mice at a dose of 5 mg/kg twice a week for 8 weeks, and the mice were executed 24 hours after the last injection, and the brain tissues of the mice were taken for western blot experiments. The results showed that HTT si-5 plasmid molecule could effectively reduce the protein and mRNA expression levels of mHTT in brain tissues in vivo (as shown in Figure 5, A-C). The aggregation of mHTT mutant proteins in the cortex and striatum was reduced (as shown in Figure 5E, EM48 is the antibody that specifically binds to the aggregated mHTT).

Moreover, HTT si-1, 2, 3, and 4 plasmid molecules were also effective in reducing the expression level of mHTT in brain tissue in vivo.

### Example 3: Effect of HTT siRNA/RVG plasmid on the improvement of motor ability in HD transgenic mice

HTT si-5 was used as an example for detailed explanation. The locomotor ability of HD transgenic mice was also evaluated by performing a fatigue rotarod experiment on N171-82Q mice prior to drug administration, with one day of training, followed by three times a day for three days, and completing a three-day fatigue rotarod experiment as well as a balance experiment after drug administration. The results showed that the injection of HTT siRNA/RVG plasmid could effectively improve the fatigue tolerance of N171-82Q mice.

In YAC128 mice, a three-day fatigue rotarod test was performed prior to drug administration and again after four and eight weeks of tail vein injection. The results showed that injection of HTT siRNA/RVG plasmid could effectively alleviate the deterioration of fatigue rotarod test index in YAC128 mice (shown in Figure 5D).

Moreover, injection of HTT siRNA(1-4)/RVG plasmid was also effective in alleviating the deterioration of the fatigue rod test in YAC128 mice.

All literature mentioned in the present invention is cited as references in this application as if each literature were cited separately as a reference. Furthermore it should be understood that after reading the above disclosure of the present invention, various alterations or modifications can be made to the present invention by those skilled in the art, and these equivalent forms fall within the scope defined by the claims of this application.

## Claims

1. A precursor sequence having a structure from 5' to 3' as shown in formula (I): 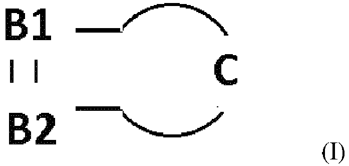
wherein B1 is a desired first ribonucleic acid sequence, wherein said first ribonucleic acid sequence comprises a HTT siRNA sense-strand sequence,
B2 is a sequence substantially or completely complementary to B1, and B2 is not complementary to C,
C is a stem-loop sequence;
wherein said HTT siRNA sense-strand sequence has a nucleotide sequence selected from the group consisting of: SEQ ID NO: 1, SEQ ID NO: 2, SEQ ID NO: 3, SEQ ID NO: 4, SEQ ID NO.: 5, or a combination thereof.

2. The precursor sequence as claimed in Claim 1, **characterized in that** said precursor sequence has a sequence as shown in any of SEQ ID NO.:6-10.

3. A polynucleotide, **characterized in that** said polynucleotide is capable of being transcribed by a host to form the precursor sequence as defined in Claim 1.

4. An expression vector, **characterized in that** said expression vector contains the precursor sequence as defined in Claim 1 or the polynucleotide as defined in Claim 3.

5. A pharmaceutical formulation, **characterized in that** said formulation comprises:
(a) an expression vector for expressing an siRNA that inhibits the expression of a HTT gene; and
(b) a pharmaceutically acceptable vector.

6. The pharmaceutical formulation as claimed in claim 5, **characterized in that** said expression vector expresses the precursor sequence as shown in formula I: 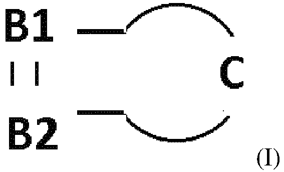
wherein B1 is a desired first ribonucleic acid sequence, wherein said first ribonucleic acid sequence comprises a HTT siRNA sense-strand sequence,
B2 is a sequence substantially or completely complementary to B1, and B2 is not complementary to C,
C is a stem-loop sequence;
wherein said HTT siRNA sense-strand sequence has a nucleotide sequence selected from the group consisting of: SEQ ID NO: 1, SEQ ID NO: 2, SEQ ID NO: 3, SEQ ID NO: 4, SEQ ID NO.: 5, or a combination thereof.

7. A siRNA for inhibiting the expression of a HTT gene, **characterized in that** said siRNA has a sense strand nucleotide sequence selected from the group consisting of: SEQ ID NO: 1, 2, 3, 4, 5, or a combination thereof.

8. A pharmaceutical composition, **characterized in that** said pharmaceutical composition comprises the precursor sequence of Claim 1, or an expression vector of Claim 4, and a pharmaceutically acceptable vector.

9. The use of the siRNA as claimed in Claim 7, the precursor sequence as claimed in Claim 1, or the expression vector as claimed in Claim 4, **characterized in that** the use is for the preparation of a composition or formulation, said composition or formulation being used for (i) treating Huntington's disease.

10. The use as claimed in Claim 9, **characterized in that** said composition or formulation is further used for one or more purposes selected from the group consisting of:
(ii) inhibition of HTT expression in mammalian brain tissue; and/or
(iii) improving motor coordination in mammals.
